# FASCICULE DE BREVET EUROPEEN

(11) **EP 0 951 704 B1**
(45) Date de publication et mention de la délivrance du brevet: **17.10.2001**
(21) Numéro de dépôt: 98902039.1
(22) Date de dépôt: 07.01.1998
(51) Int. Cl.: G08B 21/00

(54) **DISPOSITIF DE SURVEILLANCE DE POLLUTION AUTOMOBILE EN ZONE URBAINE**
VORRICHTUNG ZUR ÜBERWACHUNG VON UMWELTVERSCHMUTZUNG DURCH FAHRZEUGE IN STÄDTISCHEN BEREICHEN
DEVICE FOR MONITORING POLLUTION CAUSED BY MOTOR VEHICLES IN AN URBAN AREA

(30) Priorité: 09.01.1997 FR 9700237
(43) Date de publication de la demande: 27.10.1999
(73) Titulaire: SCHLUMBERGER Systèmes, 92120 Montrouge (FR)
(72) Inventeur: BRUSSEAUX, Thierry, F-25720 Avenay (FR); FLINOIS, Xavier, F-75017 Paris (FR)
(74) Mandataire: Lemoyne, Didier
(86) Numéro de dépôt international: FR9800022
(87) Numéro de publication internationale: WO9830990

(56) Documents cités:
- EP-A- 0 527 307
- EP-A- 0 622 625

## Description

La présente invention concerne un dispositif de surveillance de pollution générée par les véhicules automobiles dans une zone urbaine.

L'invention trouve une application particulièrement avantageuse dans le domaine de la détection et de la prévention de la pollution automobile en milieu urbain, que cette pollution soit due à la présence de polluant chimique, type gaz d'échappement, dans l'air ambiant ou au bruit produit par le moteur et le roulement des véhicules.

Aujourd'hui, l'augmentation constante de la circulation automobile dans le centre des grandes agglomérations provoque une pollution de l'atmosphère qui, par exemple, peut atteindre dans certains cas un niveau tel qu'elle met en danger la santé des personnes.

Déjà, certaines municipalités se sont émues de ce problème et ont cherché à mettre en place des dispositifs de surveillance de la qualité de l'air. Ces dispositifs sont en général des armoires techniques munies de détecteurs placés en hauteur et aptes à mesurer la concentration des gaz polluants pouvant se rencontrer en atmosphère urbaine. Outre qu'ils sont très volumineux, complexes et onéreux, les dispositifs de surveillance connus présentent l'inconvénient qu'ils sont très peu nombreux, 1 à 10 dans une même ville, et ne peuvent donc refléter avec précision la répartition géographique de la pollution. D'autre part, ces dispositifs mesurent une pollution globale, sans distinction de son origine. En particulier, il n'est pas possible de déterminer, notamment à cause de la situation en hauteur des détecteurs, la pollution effectivement ressentie par les personnes circulant en ville et la part de la pollution générée par les véhicules automobiles. Enfin, les dispositifs de surveillance actuels ne font qu'enregistrer au cours de la journée les mesures de concentration de polluants en vue d'un traitement centralisé en temps différé sans possibilité de réaction en temps réel dans un délai admissible en cas de pics de pollution.

Aussi, le problème technique à résoudre par l'objet de la présente invention est de proposer un dispositif de surveillance de pollution générée par les véhicules automobiles dans une zone urbaine, dispositif qui permettrait, de manière simple et peu coûteuse, d'établir une répartition précise de la pollution automobile dans la zone considérée et d'offrir des moyens de réagir en temps réel sur la circulation des véhicules pour faire diminuer la pollution aussi rapidement que possible en cas de dépassement d'un seuil déterminé.

La solution au problème technique posé consiste, selon la présente invention, en ce que, ladite zone étant équipée d'une pluralité d'éléments de mobilier urbain munis, chacun, de moyens d'acquisition et de traitement d'informations et de moyens de communication desdites informations avec un site central, ledit dispositif de surveillance comprend au moins un élément de mobilier urbain muni, en outre, au moins d'un détecteur d'une grandeur caractéristique de pollution, apte à fournir auxdits moyens d'acquisition et de traitement d'informations une mesure de ladite grandeur.

En particulier, selon un mode de mise en oeuvre de l'invention, lesdits éléments de mobilier urbain sont des horodateurs.

De même, l'invention prévoit que ladite grandeur caractéristique de pollution est constituée par la concentration d'un polluant chimique dans l'air ambiant et/ou le niveau sonore dû au bruit produit par lesdits véhicules automobiles.

Le dispositif de surveillance conforme à l'invention présente de nombreux avantages. D'une part il utilise une infrastructure de mobilier urbain déjà existante, ce qui se traduit par un coût relativement faible, représenté essentiellement par l'installation d'un détecteur de polluant et/ou d'un transducteur acoustique, et l'adaptation des moyens d'acquisition et de traitement d'informations par une simple modification de logiciels. D'autre part, les éléments de mobilier urbain, tels que les horodateurs, offrent une implantation géographique dense permettant d'obtenir une connaissance précise de la répartition de la pollution, même si on se contente par exemple de n'équiper qu'un ou deux horodateurs dans une même rue. De plus, les horodateurs, de même que la plupart des éléments de mobilier urbain, sont idéalement placés en hauteur pour détecter la pollution aux endroits où elle se fait le plus sentir et pour évaluer de façon prédominante la pollution d'origine automobile. Par ailleurs, le fait que lesdits éléments de mobilier urbain soient reliés en réseau à un site central permet, sans modification sensible, de centraliser les mesures de pollution effectuées par un traitement différé en vue d'établir une cartographie exacte, rue par rue, de la pollution au cours de la journée ou de la semaine, toutes ces informations étant d'une grande utilité pour définir un meilleur plan de circulation du point de vue de la pollution automobile.

L'invention prévoit en outre que les moyens d'acquisition et de traitement d'informations comprennent des moyens de calcul de stockage d'une pluralité de mesures échantillonnées de la grandeur caractéristique de pollution et des moyens de calcul de paramètres représentatifs de ladite pollution. L'élément de mobilier urbain concerné peut donc fournir à intervalles de temps réguliers lesdits paramètres représentatifs, telles que moyennes horaires glissantes, en rapport avec les prescriptions officielles.

Enfin, selon une autre caractéristique avantageuse de l'invention, les moyens d'acquisition et de traitement d'informations sont aptes à établir un message d'alarme lorsqu'au moins un desdits paramètres représentatifs de pollution dépasse un seuil déterminé. Cette information de dépassement est transmise en temps réel aux autorités via le site central pour information et prise éventuelle de décisions. On peut également envisager une réaction automatique du dispositif de surveillance lorsque, selon l'invention, ledit site central dispose de moyens de commande, en cas de dépassement dudit seuil, d'équipements urbains de prévention, avec pour effet de bloquer l'accès à certaines rues ou certains secteurs par la mise en oeuvre de feux tricolores, de panneaux indicateurs, de bornes rétractables, de barrières mobiles, ou d'informer les automobilistes au moyen de panneaux à message variable, les invitant à stopper leur véhicule, voire même à les diriger vers des zones de stationnement dont le tarif pourra être momentanément réduit.

La description qui va suivre en regard du dessin annexé, donné à titre d'exemple non limitatif, fera bien comprendre en quoi consiste l'invention et comment elle peut être réalisée.

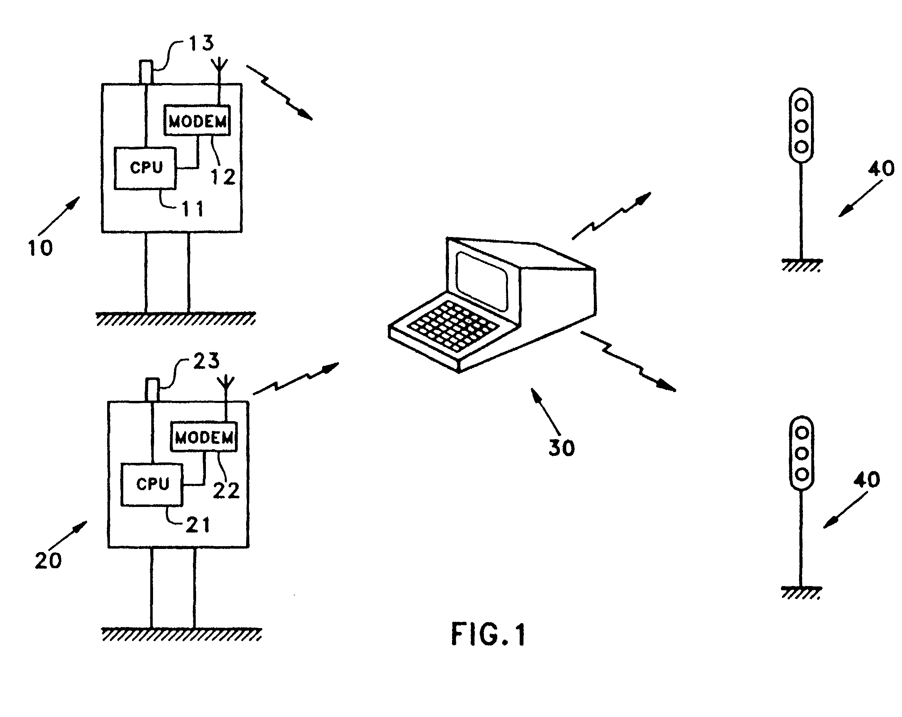

La figure 1 est un schéma d'un dispositif de surveillance conforme à l'invention.

Sur la figure 1 est représenté schématiquement un dispositif de surveillance de pollution générée par les véhicules automobiles dans une zone urbaine.

Ce dispositif comporte des éléments de mobilier urbain, représenté ici par deux horodateurs 10, 20, situés dans la zone urbaine dont la pollution automobile doit être surveillée.

Les horodateurs 10, 20 sont équipés d'origine de moyens 11, 21, d'acquisition et de traitement d'informations, constitués par une unité centrale chargée de la gestion de l'horodateur correspondant, ainsi que des moyens 12, 22 de communication avec un site central 30 qui, dans l'exemple de réalisation de la figure 1, sont des modems destinés à réaliser une liaison hertzienne avec ledit central 30, ladite liaison hertzienne pouvant d'ailleurs tout aussi bien être remplacée par une liaison téléphonique ou autre. Relativement aux horodateurs 10, 20, les unités centrales 11, 21 sont prévues notamment pour détecter les anomalies de fonctionnement, comme des pannes de certains organes ou encore la fin du rouleau de tickets. Ces informations sont ensuite dirigées vers le site central 30 pour intervention du service de maintenance, par l'intermédiaire des modems 12, 22.

Comme le montre la figure 1, les horodateurs 10, 20 sont munis, en outre, de détecteurs 13, 23 d'un polluant chimique caractéristique de la pollution automobile, par exemple le monoxyde de carbone CO. De manière connue, les détecteurs correspondants peuvent être des capteurs infrarouge, électrochimiques ou électriques aptes à mesurer les variations de conductivité d'une pastille de SiO₂. Bien entendu, il serait tout à fait possible de choisir d'autres polluants automobiles, tels que les oxydes d'azote NO, NO₂, etc.

On comprendra que la référence faite ici à la seule pollution chimique n'est nullement limitative et que l'invention s'étend également à la pollution sonore provoquée par la circulation automobile, les horodateurs 10 et 20 étant équipés, éventuellement en combinaison avec les détecteurs 13, 23, de transducteurs acoustiques, tels que des microphones, dont la relation aux autres organes des horodateurs est en tout point identique à celle qui va maintenant être décrite en liaison avec les détecteurs 13, 23 de polluant chimique.

Les détecteurs 13, 23 sont reliés aux unités centrales 11, 21 de manière à leur transmettre en continu la valeur mesurée de la concentration dudit polluant dans l'air ambiant qui est la grandeur caractéristique de la pollution d'origine chimique. En conséquence, lesdites unités centrales sont organisées de manière à recevoir les mesures provenant des détecteurs 13, 23 dans des moyens de stockage, du type base de données, dans lesquels sont inscrites lesdites mesures selon un pas d'échantillonnage donné, par exemple toutes les minutes. Ces mesures échantillonnées sont transmises au site central 30 pour traitement en différé afin d'établir notamment une cartographie précise de la pollution automobile dans la zone considérée, les horodateurs 10, 20 étant placés à des endroits judicieusement choisis. Comme mentionné plus haut, ce type d'informations peut permettre aux autorités responsables de définir des plans de circulation de moindre pollution.

A l'inverse de ce traitement différé visant une gestion à long terme de la pollution automobile, le dispositif de surveillance, objet de l'invention, offre la possibilité de réagir en temps réel en cas de pointes de pollution. A cet effet, les unités centrales 11, 21 comprennent, outre lesdits moyens de stockage, des moyens de calcul de paramètres représentatifs de la pollution en conformité avec les prescriptions officielles. A titre d'exemple, le paramètre calculé par lesdites unités centrales peut être une moyenne glissante sur une période donnée, quart d'heure ou heure, des mesures de concentration de polluant effectuées toutes les minutes.

Ces paramètres représentatifs sont comparés en permanence à des seuils prédéterminés de manière à pouvoir, en cas de dépassement, établir un message d'alarme à destination du site central 30. Comme chaque horodateur 10, 20 est identifié individuellement au niveau dudit site central, il est toujours possible de connaître l'origine géographique de l'alarme et donc de prendre des mesures de prévention appropriées. Dans ce but, le site central 30 dispose de moyens de commande, tels qu'une liaison hertzienne par exemple, destinés à mettre en oeuvre, lorsqu'une alarme est reçue, des équipements urbains 40 afin de réglementer la circulation automobile dans le secteur où le pic de pollution a été détecté. Les équipements urbains concernés peuvent être des feux rouges, comme illustré sur la figure 1, mais également des barrières, des bornes rétractables ou des panneaux d'informations recommandant aux automobilistes d'arrêter leur véhicule, au besoin dans des parcs de stationnement dont le tarif aura été éventuellement modifié.

## Revendications

1. Dispositif de surveillance de pollution générée par les véhicules automobiles dans une zone urbaine, **caractérisé en ce que**, ladite zone étant équipée d'une pluralité d'éléments de mobiliers urbains munis, chacun, de moyens (11, 21) d'acquisition et de traitement d'informations et de moyens (12, 22) de communication desdites informations avec un site central (30), ledit dispositif de surveillance comprend au moins un élément (10, 20) de mobilier urbain muni, en outre, au moins d'un détecteur (13, 23) d'une grandeur caractéristique de pollution, apte à fournir auxdits moyens (11, 21) d'acquisition et de traitement d'informations une mesure de ladite grandeur.

2. Dispositif de surveillance selon la revendication 1, **caractérisé en ce que** les moyens (11, 21) d'acquisition et de traitement d'informations comprennent des moyens de stockage d'une pluralité de mesures échantillonnées de la grandeur caractéristique de pollution et des moyens de calcul de paramètres représentatifs de ladite pollution.

3. Dispositif de surveillance selon la revendication 2, **caractérisé en ce que** les moyens (11, 21) d'acquisition et de traitement d'informations sont aptes à établir un message d'alarme lorsqu'au moins un desdits paramètres représentatifs de pollution dépasse un seuil déterminé.

4. Dispositif de surveillance selon la revendication 3, **caractérisé en ce que** ledit site central (30) dispose de moyens de commande, en cas de dépassement dudit seuil, d'équipements urbains (40) de prévention.

5. Dispositif de surveillance selon l'une quelconque des revendications 1 à 4, **caractérisé en ce que** lesdits éléments de mobilier urbain sont des horodateurs (10, 20).

6. Dispositif de surveillance selon l'une quelconque des revendications 1 à 5, **caractérisé en ce que** ladite grandeur caractéristique de pollution est constituée par la concentration d'un polluant chimique dans l'air ambiant et/ou le niveau sonore dû au bruit produit par lesdits véhicules automobiles.

## Claims

1. Monitoring apparatus for monitoring pollution generated by motor vehicles in an urban area, said apparatus being **characterized in that**, with said area being equipped with a plurality of items of street furniture, each of which is provided with data acquisition and processing means (11, 21) for acquiring and processing data, and with communications means (12, 22) for communicating said data to a central site (30), said monitoring apparatus includes at least one item of street furniture (10, 20) further provided with at least one detector (13, 23) for detecting a magnitude characteristic of pollution, and suitable for delivering a measurement of said magnitude to said data acquisition and processing means (11, 21).

2. Monitoring apparatus according to claim 1, **characterized in that** the data acquisition and processing means (11, 21) comprise storage means for storing a plurality of sampled measurements of the magnitude characteristic of pollution, and computation means for computing parameters representative of said pollution.

3. Monitoring apparatus according to claim 2, **characterized in that** the data acquisition and processing means (11, 21) are suitable for establishing an alarm message when at least one of said parameters representative of pollution exceeds a determined threshold.

4. Monitoring apparatus according to claim 3, **characterized in that** said central site (30) is provided with control means serving, in the event that said. threshold is exceeded, to control street furniture for prevention purposes (40).

5. Monitoring apparatus according to any one of claims 1 to 4, **characterized in that** said items of street furniture are pay-and-display parking meters (10, 20).

6. Monitoring apparatus according to any one of claims 1 to 5, **characterized in that** said magnitude characteristic of pollution is constituted by the concentration of a chemical pollutant in ambient air and/or the sound level due to noise produced by said motor vehicles.

## Patentansprüche

1. Vorrichtung zur Überwachung der durch Kraftfahrzeuge in einem Stadtgebiet generierten Luftverschmutzung und Lärmbelästigung, **dadurch gekennzeichnet, daß** besagtes Gebiet mit einer Vielzahl mobiler urbaner Elementen ausgerüstet ist, von denen jedes mit Mitteln (11, 21) zum Erfassen und zur Verarbeitung von Informationen und Mitteln (12, 22) zur Kommunikation besagter Informationen an eine zentralen Anlage (30) ausgerüstet ist, besagte Vorrichtung zur Überwachung umfaßt wenigstens ein mobiles urbanes Element (10, 20), das unter anderem mit wenigstens einem Sensor (13, 23) einer charakteristischen Größe für die Luftverschmutzung oder Lärmbelästigung ausgerüstet und geeignet ist, besagten Mitteln (11, 21) zum Erfassen und zur Verarbeitung von Informationen ein Maß besagter Größe zu liefern.

2. Vorrichtung zur Überwachung gemäß Anspruch 1, **dadurch gekennzeichnet, daß** die Mittel (11, 21) zum Erfassen und zur Verarbeitung von Informationen Mittel zur Speicherung einer Vielzahl von geeichten Maßen der charakteristischen Größe für Luftverschmutzung und Lärmbelästigung und Mittel zur Berechnung der repräsentativen Parameter besagter Luftverschmutzung und Lärmbelästigung darstellen.

3. Vorrichtung zur Überwachung gemäß Anspruch 2, **dadurch gekennzeichnet, daß** die Mittel (11, 21) zum Erfassen und zur Verarbeitung von Informationen geeignet sind, eine Alarmmeldung auszugeben, wenn wenigstens einer der besagten repräsentativen Parameter für Luftverschmutzung und Lärmbelästigung einen vorgegebenen Grenzwert überschreitet.

4. Vorrichtung zur Überwachung gemäß Anspruch 3, **dadurch gekennzeichnet, daß** besagte zentrale Einheit (30) über Steuermittel verfügt, im Falle des Überschreitens des besagten Grenzwertes, über urbane Präventionsausrüstungen (40).

5. Vorrichtung zur Überwachung gemäß Anspruch 1 bis 4, **dadurch gekennzeichnet, daß** die besagten mobilen urbanen Elemente Zeitstempler (10, 20) sind.

6. Vorrichtung zur Überwachung gemäß Anspruch 1 bis 5, **dadurch gekennzeichnet, daß** die besagte charakteristische Größe für Luftverschmutzung und Lärmbelästigung durch die Konzentration einer chemischen Verschmutzungs- und Lärmbelästigungsursache in der Umgebungsluft und / oder den Lärmpegel des von Kraftfahrzeugen produzierten Lärms gebildet wird.
